# EUROPEAN PATENT APPLICATION

(11) **EP 4 206 270 A1**
(43) Date of publication of application: **05.07.2023**
(21) Application number: 22846149.7
(22) Date of filing: 14.07.2022
(51) Int. Cl.: C08J 11/24, C07C 63/26, C07C 51/09, C07C 51/42, C08G 63/181, C08G 63/183, C08K 5/00, C08K 5/10

(54) **MONOMER COMPOSITION FOR SYNTHESIZING RECYCLED PLASTIC, METHOD FOR PREPARING SAME, AND RECYCLED PLASTIC, MOLDED ARTICLE, AND PLASTICIZER COMPOSITION USING SAME**

(30) Priority: 19.07.2021 KR 20210094470; 19.07.2021 KR 20210094471; 19.07.2021 KR 20210094472; 19.07.2021 KR 20210094473
(71) Applicant: Lg Chem, Ltd., Seoul 07336 (KR)
(72) Inventor: KIM, Jeongnam, Daejeon 34122 (KR); PARK, Tae Seung, Daejeon 34122 (KR); HWANG, Jihwan, Daejeon 34122 (KR); LEE, Kang Geun, Daejeon 34122 (KR); KIM, Byoung Hyoun, Daejeon 34122 (KR); HONG, Mooho, Daejeon 34122 (KR)
(74) Representative: Goddar, Heinz J.
(86) International application number: PCT/KR2022/010305
(87) International publication number: WO 2023/003279

(57) **Abstract**

The present invention relates to a monomer composition for synthesizing recycled plastic which is extremely reduced in the content of organic impurities such as formic acid and acetic acid while recovering from a (co)polymer synthesized from monomers containing terephthalic acid, a method for preparing the same, and a recycled plastic, molded product, and plasticizer composition using the same.

## Description

### [TECHNICAL FIELD]

### CROSS-REFERENCE TO RELATED APPLICATION(S)

This application claims the benefit of Korean Patent Application No. 10-2021-0094470 filed on July 19, 2021, Korean Patent Application No. 10-2021-0094471 filed on July 19, 2021, Korean Patent Application No. 10-2021-0094472 filed on July 19, 2021 and Korean Patent Application No. 10-2021-0094473 filed on July 19, 2021 in the Korean Intellectual Property Office, the contents of which are incorporated herein by reference in their entirety.

The present invention relates to a monomer composition for synthesizing recycled plastic that is capable of remarkably reducing the content of organic impurities and thus securing high-purity terephthalic acid, when recovering terephthalic acid through a depolymerization reaction of a (co)polymer synthesized from a monomer containing terephthalic acid, a method for preparing the same, and a recycled plastic, molded product, and plasticizer composition using the same.

### [BACKGROUND ART]

Polyethylene terephthalate (PET) is a thermoplastic (co)polymer that has excellent characteristics such as excellent transparency and heat insulation property, and is a plastic widely used in electric wire coverings, daily necessities, toys, electrical insulators, radios, television cases, packaging materials, and the like.

Although polyethylene terephthalate is widely used for various purposes, environmental and health concerns during waste treatment have been continuously raised. Currently, a physical recycling method is being carried out, but in this case, a problem accompanying the deterioration of the quality occurs, and thus, research on the chemical recycling of polyethylene terephthalate are underway.

Terephthalic acid is a useful compound used as a raw material for various kinds of products, which is used as a main raw material for polyethylene terephthalate (PET), polyester fibers, and polyester films for packaging and containers.

Subjecting ethylene glycol together with terephthalic acid to a polycondensation to produce polyethylene terephthalate (PET) is a reversible reaction process, and polyethylene terephthalate (PET) can be depolymerized and recycled to a monomer or oligomer.

Various methods have been conventionally proposed as a method for decomposing polyethylene terephthalate (PET) to recover a monomer as a raw material. Monomers that can be used in the polycondensation reaction for re-producing into a recycled plastic can be obtained through alkali decomposition of polyethylene terephthalate (PET) waste.

For example, polyethylene terephthalate (PET) decomposition products under basic conditions include salts of ethylene glycol and terephthalic acid, and a salt of terephthalic acid is further subjected to a neutralization reaction with a strong acid to prepare terephthalic acid.

However, other organic impurities are detected in terephthalic acid obtained by the existing method and thus, when it is reused as a raw material for producing high value-added plastics such as PBT/TPEE, there is a limit in that the reaction time is slow and the color quality is deteriorated.

Therefore, in the process of decomposing the (co)polymer synthesized from the monomer containing terephthalic acid including polyethylene terephthalate (PET) and recovering the monomer as a raw material, there is a need to develop a method that can significantly reduce the content of organic impurities.

### [DETAILED DESCRIPTION OF THE INVENTION]

### [Technical Problem]

It is an object of the present invention to provide a monomer composition for synthesizing recycled plastic that is capable of remarkably reducing the content of organic impurities and thus securing high-purity terephthalic acid, when recovering terephthalic acid through a depolymerization reaction of a (co)polymer synthesized from a monomer containing terephthalic acid.

It is another object of the present invention to provide a method for preparing a monomer composition for synthesizing recycled plastic, and a recycled plastic, molded product, and plasticizer composition using the monomer composition for synthesizing recycled plastic.

### [Technical Solution]

In order to achieve the above object, provided herein is a monomer composition for synthesizing recycled plastic, which comprises terephthalic acid, and which further comprises one or more organic impurities selected from the group consisting of formic acid and acetic acid, wherein a weight ratio of the organic impurities measured using a gas chromatography mass spectrometry is less than 5 ug relative to 1 g of the monomer composition for synthesizing recycled plastic, and wherein the monomer composition for synthesizing recycled plastic is recovered from a (co)polymer synthesized from a monomer containing terephthalic acid.

Also provided herein is a method for preparing a monomer composition for synthesizing recycled plastic, comprising the steps of: subjecting a (co)polymer synthesized from a monomer containing terephthalic acid to a depolymerization reaction and removing a diol component; washing the depolymerization reaction product from which the diol component has been removed with a solvent at a temperature of 20°C or more and 100°C or less; and dissolving the washed depolymerization reaction product in water and then recrystallizing it, wherein the step of dissolving the washed depolymerization reaction product in water and then recrystallizing it comprises dissolving the washed depolymerization reaction product in water at a temperature of 200°C to 300°C; and cooling the dissolved solution to a temperature lower by 50°C to 200°C than the solution temperature.

Further provided herein is a recycled plastic comprising a reaction product of the monomer composition for synthesizing recycled plastic and a comonomer.

Further provided herein is a molded product comprising the recycled plastic.

Further provided herein is a plasticizer composition comprising a reaction product of the monomer composition for synthesizing recycled plastic and an alcohol.

Below, a monomer composition for synthesizing recycled plastic, a method for preparing the same, and a recycled plastic, molded product, and plasticizer composition using the same according to specific embodiments of the present invention will be described in more detail.

Unless explicitly stated herein, the technical terms used herein are for the purpose of describing specific embodiments only and is not intended to limit the scope of the invention.

The singular forms "a," "an" and "the" used herein are intended to include plural forms, unless the context clearly indicates otherwise.

It should be understood that the terms "comprise," "include", "have", etc. are used herein to specify the presence of stated feature, region, integer, step, action, element and/or component, but do not preclude the presence or addition of one or more other feature, region, integer, step, action, element, component and/or group.

Further, the terms including ordinal numbers such as "a first", "a second", etc. are used only for the purpose of distinguishing one component from another component, and are not limited by the ordinal numbers. For instance, a first component may be referred to as a second component, or similarly, the second component may be referred to as the first component, without departing from the scope of the present invention.

In the present specification, the (co)polymer includes both a polymer and a copolymer, the polymer means a homopolymer composed of a single repeating unit, and the copolymer means a composite polymer containing two or more types of repeating units.

### 1. Monomer Composition for Synthesizing Recycled Plastic

According to one embodiment of the present invention, there can be provided a monomer composition for synthesizing recycled plastic, which comprises terephthalic acid, and which further comprises one or more organic impurities selected from the group consisting of formic acid and acetic acid, wherein a weight ratio of the organic impurities measured using a gas chromatography mass spectrometry is less than 5 ug relative to 1 g of the monomer composition for synthesizing recycled plastic, and wherein the monomer composition for synthesizing recycled plastic is recovered from a (co)polymer synthesized from a monomer containing terephthalic acid.

The present inventors has found through experiments that, despite that it is recovered from a (co)polymer synthesized from a monomer containing terephthalic acid, as in the monomer composition for synthesizing recycled plastic of the above one embodiment, the ratio of organic impurities and not terephthalic acid, which is the main synthetic target material in the present invention, is extremely reduced to less than 5 ug relative to 1 g of the monomer composition for synthesizing recycled plastic, and when synthesizing polyethylene terephthalate or high value-added plastics (PBT, TPEE) using this, the polymerization time can be shortened, the polymerization production efficiency can be improved, and excellent physical properties can be realized in terms of color quality, and completed the present invention.

In particular, the detected amount of organic impurities (formic acid, acetic acid) was significantly reduced as compared to terephthalic acid recovered from a (co)polymer synthesized from a monomer containing terephthalic acid obtained by conventional methods. However, this is considered to be because in the present invention, the organic impurities (formic acid, acetic acid) could be effectively removed by the washing step and recrystallization step that are characteristic in the preparation method of the monomer composition for synthesizing recycled plastic, which will be described later.

Additionally, in the present invention, toxic organic solvents (e.g., DMAc) and organic acids (e.g., acetic acid) are not used for purification in the preparation method of the monomer composition for synthesizing recycled plastic, and thus, purification is possible using only water unlike the conventionally known terephthalic acid purification process, and depolymerization can also be performed using water as a solvent, which has the technical advantage of being an environmentally friendly process.

Specifically, the monomer composition for synthesizing recycled plastic of the one embodiment may include terephthalic acid. The terephthalic acid is characterized by being recovered from the (co)polymer synthesized from the monomer containing terephthalic acid which is used for the recovery of the monomer composition for synthesizing recycled plastic.

That is, this means that recovery is performed from the (co)polymer synthesized from the monomer containing terephthalic acid in order to obtain the monomer composition for synthesizing recycled plastic of the one embodiment, and as a result, terephthalic acid is also obtained together. Therefore, apart from the recovery from the (co)polymer synthesized from the monomer containing terephthalic acid in order to prepare the monomer composition for synthesizing recycled plastic of the one embodiment, the case where new terephthalic acid is added from the outside is not included in the scope of terephthalic acid of the present invention.

Specifically, "recovered from the (co)polymer synthesized from the monomer containing terephthalic acid" means that it is obtained through a depolymerization reaction of the (co)polymer synthesized from the monomer containing terephthalic acid. The depolymerization reaction can be performed under acidic, neutral or basic conditions, and particularly, the depolymerization reaction can proceed under basic (alkaline) conditions.

For example, when the depolymerization reaction proceeds under the basic conditions, terephthalic acid salt called Na₂-TPA and ethylene glycol are primarily produced from the polyethylene terephthalate, and Na₂-TPA is converted to TPA through a secondary strong acid neutralization, thereby capable of recovering terephthalic acid.

That is, the terephthalic acid recovered from the (co)polymer synthesized from the monomer containing terephthalic acid may include a basic (alkaline) decomposition product of the (co)polymer synthesized from the monomer containing terephthalic acid, an acid-neutralized product thereof, or a mixture thereof. Specifically, the basic (alkaline) decomposition product of the (co)polymer synthesized from the monomer containing terephthalic acid may include Na₂-TPA, and the acid-neutralized product of the basic (alkaline) decomposition product of the (co)polymer synthesized from the monomer containing terephthalic acid may include terephthalic acid.

The terephthalic acid may have a molar ratio of more than 99.15 mol%, or 99.2 mol% or more, or 100 mol% or less, or more than 99.15 mol% and 100 mol% or less, or more than 99.2 mol% and 100 mol% or less based on 100 mol% of the total monomer compounds contained in the monomer composition for synthesizing recycled plastic.

Examples of the method for measuring the molar ratio of terephthalic acid are not particularly limited, and, for example, ¹H NMR, ICP-MS analysis, HPLC analysis, and the like can be used without limitation. As the specific methods, conditions, devices and the like of the NMR, ICP-MS and HPLC, conventionally well-known various contents can be applied without limitation.

In an example of the method for measuring the molar ratio of terephthalic acid, 5 mg or more and 20 mg or less of the monomer composition for synthesizing recycled plastic is collected as a sample under the conditions of normal pressure, 20°C or more and 30°C or less, dissolved in 1 ml of DMSO-d6 solvent, and then ¹H NMR spectrum is obtained via an Agilent DD1 500 MHz NMR instrument, and by using the analysis software (MestReC), the detected peaks of all materials such as terephthalic acid (TPA), isophthalic acid (IPA) and the like are respectively assigned, integrated, and the molar ratio (mol%) of terephthalic acid contained in 100 mol% of the total monomer compounds analyzed in the sample is calculated based on the integral values of the peaks.

In this manner, the ratio of terephthalic acid, which is the main synthetic target material in the present invention, is extremely increased to more than 99.15 mol% based on 100 mol% of the total monomer compounds contained in the monomer composition for synthesizing recycled plastic, and terephthalic acid or impurity monomers (e.g., isophthalic acid) are minimized, thereby capable of realizing excellent physical properties in the synthesis of polyethylene terephthalate or high value-added plastics (PBT, TPEE).

Further, the monomer composition for synthesizing recycled plastic of the one embodiment may further include isophthalic acid having a molar ratio of less than 0.85 mol% based on 100 mol% of the total monomer compounds contained in the monomer composition for synthesizing recycled plastic.

The isophthalic acid is characterized by being recovered from the (co)polymer synthesized from the monomer containing terephthalic acid that is used for the recovery of the monomer composition for synthesizing recycled plastic.

That is, this means that recovery is performed from the (co)polymer synthesized from the monomer containing terephthalic acid in order to obtain the monomer composition for synthesizing recycled plastic of the one embodiment, and as a result, isophthalic acid is also obtained together. Therefore, apart from the recovery from the (co)polymer synthesized from the monomer containing terephthalic acid in order to prepare the monomer composition for synthesizing recycled plastic of the one embodiment, the case where new isophthalic acid is added from the outside is not included in the scope of isophthalic acid of the present invention.

Specifically, "recovered from the (co)polymer synthesized from the monomer containing terephthalic acid" means that it is obtained through a depolymerization reaction of the (co)polymer synthesized from the monomer containing terephthalic acid. The depolymerization reaction can be carried out under acidic, neutral or basic conditions, and particularly, the depolymerization reaction can proceed under basic (alkaline) conditions.

The terephthalic acid may have a molar ratio of less than 0.85 mol%, or less than 0.5 mol%, or 0.1 mol% or less, or 0 mol% or more and less than 0.85 mol%, or 0 mol% or more and 0.5 mol% or less, or 0 mol% or more and 0.1 mol% or less based on 100 mol% of the total monomer compounds contained in the monomer composition for synthesizing recycled plastic.

Examples of the method for measuring the molar ratio of terephthalic acid are not particularly limited, and, for example, ¹H NMR, ICP-MS analysis, HPLC analysis, and the like can be used without limitation. As for the specific methods, conditions, devices and the like of the NMR, ICP-MS and HPLC, conventionally well-known various contents can be applied without limitation.

In one example of the method for measuring the molar ratio of terephthalic acid, 5 mg or more and 20 mg or less of the monomer composition for synthesizing recycled plastic is collected as a sample under the conditions of normal pressure, 20°C or more and 30°C or less, dissolved in 1 ml DMSO-d6 solvent, and then ¹H NMR spectrum is obtained via an Agilent DD1 500 MHz NMR instrument, and by using the analysis software (MestReC), the detected peaks of all materials such as terephthalic acid (TPA), isophthalic acid (IPA) and the like are respectively assigned, integrated, and the molar ratio (mol%) of terephthalic acid contained in 100 mol% of all the monomer compounds analyzed in the sample is calculated based on the integral values of the peaks.

In this manner, the ratio of isophthalic acid, which is an impurity monomer and not terephthalic acid, which is the main synthesis target material in the present invention is extremely decreased to 0.85 mol% or less based on 100 mol% of the total monomer compounds contained in the monomer composition for synthesizing recycled plastic, thereby capable of realizing excellent physical properties in the synthesis of polyethylene terephthalate or high value-added plastics (PBT, TPEE) using this.

Further, the monomer composition for synthesizing recycled plastic of the one embodiment is characterized by being recovered from a (co)polymer synthesized from a monomer containing terephthalic acid. That is, this means that recovery is performed from the (co)polymer synthesized from the monomer containing terephthalic acid in order to obtain the monomer composition for synthesizing recycled plastic of the one embodiment, and as a result, a monomer composition for synthesizing recycled plastic containing terephthalic acid and isophthalic acid is obtained together.

In the (co)polymer synthesized from the monomer containing terephthalic acid, the (co)polymer includes both a polymer and a copolymer, and collectively refers to a reaction product obtained from the (co)polymerization of monomers. The (co)polymer may include all low molecular weight compounds, oligomers, and polymers depending on the molecular weight range.

The (co)polymer synthesized from the monomer containing terephthalic acid may include at least one (co)polymer selected from the group consisting of polyalkylene terephthalate, polyalkylene terephthalate-based copolymer, and thermoplastic polyester elastomer. That is, the (co)polymer synthesized from the monomer containing terephthalic acid may include one type of polyalkylene terephthalate, one type of polyalkylene terephthalate-based copolymer, one type of thermoplastic polyester elastomer, or a mixture of two or more thereof.

The polyalkylene terephthalate-based copolymer means a copolymer obtained by further reacting an additional comonomer based on alkylene glycol and terephthalic acid which are monomers for synthesizing polyalkylene terephthalate.

The (co)polymer synthesized from the monomer containing terephthalic acid may include a reaction product of terephthalic acid and a comonomer. That is, the monomer containing terephthalic acid may further include a comonomer together with terephthalic acid.

Examples of the comonomer capable of reacting with terephthalic acid are also not particularly limited, and specific examples thereof may include aliphatic diols, polyalkylene oxides, fatty acids, fatty acid derivatives, or combinations thereof.

As the aliphatic diol, for example, a diol having a number average molecular weight (Mn) of 300 g/mol or less, i.e., at least one selected among ethylene glycol, propylene glycol, 1,2-propanediol, 1,3-propanediol, 1,4-butanediol (1,4-BG), 1,5-pentanediol, 1,6-hexanediol, 1,4-cyclohexanedimethanol (1,4-CHDM) can be used. As a specific example, 1,4-butanediol, ethylene glycol, 1,4-cyclohexanedimethanol, or a mixture thereof can be used.

The polyalkylene oxide is a unit constituting a soft fraction, and may include an aliphatic polyether as a constituent component. As an example, at least one selected among polyoxyethylene glycol, polypropylene glycol, poly(tetramethylene ether)glycol (PTMEG), polyoxyhexamethylene glycol, a copolymer of ethylene oxide and propylene oxide, an ethylene oxide addition polymer of polypropylene oxide glycol, and a copolymer of ethylene oxide and tetrahydrofuran can be used, and as a specific example, PTMEG can be used. In particular, PTMEG having a number average molecular weight (Mn) of 600 g/mol to 3,000 g/mol, 1,000 g/mol to 2,500 g/mol, or 1,500 g/mol to 2,200 g/mol can be used.

The fatty acid may be at least one type of aliphatic carboxylic acid compounds excluding terephthalic acid as an example, and adipic acid can be used as a specific example. The fatty acid derivative is a compound derived from the above-mentioned fatty acid, and as an example, at least one selected among fatty acid ester, fatty acid chloride, fatty acid anhydride, and fatty acid amide can be used, and as a specific example, adipic acid ester can be used.

In a more specific example, when 1,4-butanediol, which is the aliphatic diol, is used as a comonomer capable of reacting with the terephthalic acid, polybutylene terephthalate (PBT), which is a type of polyalkylene terephthalate, can be obtained by polymerization reaction of terephthalic acid and 1,4-butanediol.

Further, when ethylene glycol, which is the aliphatic diol, is used as a comonomer capable of reacting with the terephthalic acid, polyethylene terephthalate (PET), which is a type of the polyalkylene terephthalate, can be obtained through a polymerization reaction of terephthalic acid and ethylene glycol.

Further, when 1,4-butanediol as the aliphatic diol and PTMEG as the polyalkylene oxide are used together as a comonomer capable of reacting with the terephthalic acid, thermoplastic polyester elastomer (TPEE) can be obtained through a polymerization reaction of terephthalic acid, 1,4-butanediol, and PTMEG.

Further, when 1,4-butanediol as the aliphatic diol and adipic acid as the fatty acid are used together as a comonomer capable of reacting with terephthalic acid, polybutylene adipate terephthalate (PBAT), which is a type of the polyalkylene terephthalate-based copolymer, can be obtained through a polymerization reaction of terephthalic acid, 1,4-butanediol, and adipic acid.

Further, when ethylene glycol as the aliphatic diol and 1,4-cyclohexanedimethanol are used together as a comonomer capable of reacting with the terephthalic acid, a glycol-modified PET resin (glycol-modified polyethylene terephthalate, PETG), which is a type of the polyalkylene terephthalate-based copolymer, can be obtained through a polymerization reaction of terephthalic acid, ethylene glycol, and 1,4-cyclohexanedimethanol.

As a specific example, the polyalkylene terephthalate may include at least one (co)polymer selected among polybutylene terephthalate, polyethylene terephthalate, polycyclohexylene dimethylene terephthalate, polyethylene naphthalate, polybutylene naphthalate and polytrimethylene terephthalate.

Further, as a specific example, the polyalkylene terephthalate-based copolymer may include at least one (co)polymer selected among polybutylene adipate terephthalate (PBAT) and glycol-modified PET resin (glycol-modified polyethylene terephthalate, PETG).

Meanwhile, the monomer composition for synthesizing recycled plastic may further include one or more organic impurities selected from the group consisting of formic acid and acetic acid. That is, the monomer composition for synthesizing recycled plastic may further include organic impurities including one type of formic acid, one type of acetic acid, or a mixture of two types thereof. The formic acid and acetic acid are characterized in that they are recovered from the (co)polymer synthesized from the monomer containing terephthalic acid used for the recovery of the monomer composition for synthesizing the recycled plastic.

That is, this means that recovery is performed from the (co)polymer synthesized from the monomer containing terephthalic acid in order to obtain the monomer composition for synthesizing recycled plastic of the one embodiment, and as a result, formic acid and acetic acid are also obtained together. Therefore, apart from the recovery from the (co)polymer synthesized from the monomer containing terephthalic acid in order to prepare the monomer composition for synthesizing recycled plastic of the one embodiment, the case where new organic impurities are added from the outside is not included in the scope of organic impurities of the present invention.

Specifically, "recovered from the (co)polymer synthesized from the monomer containing terephthalic acid" means that it is obtained through a depolymerization reaction of the (co)polymer synthesized from the monomer containing terephthalic acid. The depolymerization reaction can be carried out under acidic, neutral, or basic conditions, and particularly, the depolymerization reaction can proceed under basic (alkaline) conditions.

The weight ratio of the organic impurities measured using a gas chromatography mass spectrometry may be less than 5 ug, or more than 0 ug, or 0 ug or more and 5 ug or less relative to 1 g of monomer composition for synthesizing recycled plastic. This is considered to be because, as described later, the method for preparing a monomer composition for synthesizing recycled plastic includes a step of washing the depolymerization reaction product from which the diol component has been removed with a solvent at a temperature of 20°C or more and 100°C or less; and a step of dissolving the washed depolymerization reaction product in water and then recrystallizing it, wherein the step of dissolving the washed depolymerization reaction product in water and then recrystallizing it comprises a step of dissolving the washed depolymerization reaction product in water at a temperature of 200°C to 300°C; and a step of cooling the dissolved solution to a temperature lower by 50°C to 200°C than the solution temperature.

The weight ratio of one type of formic acid, one type of acetic acid, or a mixture of two types, which are organic impurities other than terephthalic acid, which is the main synthetic target material in the present invention, is extremely reduced to less than 5 ug, or 0 ug or more, or 0 ug or more and 5 ug or less relative to 1 g of the monomer composition for synthesizing recycled plastic, and thus, when synthesizing polyethylene terephthalate or high value-added plastics (PBT, TPEE) using this, excellent physical properties can be realized.

Examples of the gas chromatography mass spectrometry for measuring the weight ratio of the organic impurities are not particularly limited. As an example, 0.5 g of a recycled terephthalic acid monomer composition is collected as a sample under the conditions of normal pressure and 20 ~ 30°C, quantitative analysis is performed using a Headspace-GC/MS (oven temperature: 150°C) device, and the weight ratio (*µg*/g) of formic acid (FA) and acetic acid (AA) contained therein is measured based on 1 g of the sample.

In this manner, as the weight ratio of the organic impurities measured by a gas chromatography mass spectrometry in the monomer composition for synthesizing recycled plastic of the present invention is extremely reduced to less than 5 ug, or 0 ug or more, or 0 ug or more and 5 ug or less relative to 1 g of the monomer composition for synthesizing recycled plastic, the polymerization time is shortened when synthesizing polyethylene terephthalate or high value-added plastics (PBT, TPEE) using the monomer composition for synthesizing recycled plastic, so that the polymerization production efficiency can be increased, and excellent physical properties can be realized in terms of color quality.

On the other hand, when the weight ratio of the organic impurities measured using a gas chromatography mass spectrometry is increased to 5 ug or more relative to 1 g of the monomer composition for synthesizing recycled plastic, the polymerization time is prolonged when synthesizing high value-added plastics (PBT, TPEE), so that the polymerization production efficiency is reduced and the yellow index is increased, which may result in poor color quality.

Meanwhile, the monomer composition for synthesizing recycled plastic may further include one or more aromatic impurities selected from the group consisting of 4-carboxybenzaldehyde, benzoic acid, and p-toluic acid. That is, the monomer composition for synthesizing recycled plastic may further include aromatic impurities including one type of 4-carboxybenzaldehyde, one type of benzoic acid, one type of p-toluic acid, or two or three types of mixtures thereof. The 4-carboxybenzaldehyde, benzoic acid, and p-toluic acid are recovered from the (co)polymer synthesized from the monomer containing terephthalic acid used for the recovery of the monomer composition for synthesizing recycled plastic.

That is, this means that recovery is performed from the (co)polymer synthesized from the monomer containing terephthalic acid in order to obtain the monomer composition for synthesizing recycled plastic of the one embodiment, and as a result, 4-carboxybenzaldehyde, benzoic acid, and p-toluic acid are also obtained together. Therefore, apart from the recovery from the (co)polymer synthesized from the monomer containing terephthalic acid in order to prepare the monomer composition for synthesizing recycled plastic of the one embodiment, the case where new aromatic impurities are added from the outside is not included in the scope of aromatic impurities of the present invention.

Specifically, "recovered from the (co)polymer synthesized from the monomer containing terephthalic acid" means that it is obtained through a depolymerization reaction of the (co)polymer synthesized from the monomer containing terephthalic acid. The depolymerization reaction can be carried out under acidic, neutral, or basic conditions, and particularly, the depolymerization reaction can be carried out under basic (alkaline) conditions.

The weight ratio of the aromatic impurities measured using a liquid chromatography (LC) may be less than 100 ug, or less than 50 ug, or less than 10 ug, or less than 1 ug, or 0 ug or more, or 0 ug or more and 100 ug or less, or 0 ug or more and 50 ug or less, or 0 ug or more and 10 ug or less, or 0 ug or more and 1 ug or less, relative to 1 g of monomer composition for synthesizing recycled plastic. This is considered to be because, as described later, the method for preparing a monomer composition for synthesizing recycled plastic includes a step of washing the depolymerization reaction product from which the diol component has been removed with a solvent at a temperature of 20°C or more and 100°C or less; and a step of dissolving the washed depolymerization reaction product in water and then recrystallizing it, wherein the step of dissolving the washed depolymerization reaction product in water and then recrystallizing it comprises a step of dissolving the washed depolymerization reaction product in water at a temperature of 200°C to 300°C; and a step of cooling the dissolved solution to a temperature lower by 50°C to 200°C than the solution temperature.

The weight ratio of one type of 4-carboxybenzaldehyde, one type of benzoic acid, one type of p-toluic acid, or a mixture of two or three types thereof, which are aromatic impurities other than terephthalic acid, which is the main synthetic target material in the present invention, is extremely reduced to less than 100 ug relative to 1 g of the monomer composition for synthesizing recycled plastic, and thus, when synthesizing polyethylene terephthalate or high value-added plastics (PBT, TPEE) using this, excellent physical properties can be realized.

Examples of liquid chromatography method for measuring the weight ratio of the aromatic impurities are not particularly limited. As an example, 0.5 g of a recycled terephthalic acid monomer composition is collected as a sample under the conditions of normal pressure and 20 ~ 30°C, analysis is performed using a liquid chromatography (LC) device, and the weight ratio (*µ*g/g) of 4-carboxybenzaldehyde (4-CBA), benzoic acid (BZA) and p-toluic acid (p-TA) contained therein is measured based on 1 g of the sample.

The monomer composition for synthesizing recycled plastic of the one embodiment may further include some small amounts of other additives and solvents, and the types of specific additives and solvents are not particularly limited, and various materials widely used in the terephthalic acid recovery step by depolymerization of a (co)polymer synthesized from a monomer containing terephthalic acid can be applied without limitation.

The monomer composition for synthesizing recycled plastic of the one embodiment can be obtained by a method for preparing a monomer composition for synthesizing recycled plastic, which will be described later. That is, the monomer composition for synthesizing recycled plastics of the one embodiment corresponds to a product obtained through various filtration, purification, washing, and drying steps, in order to secure only terephthalic acid in high purity, which is the main synthetic target material in the present invention, after depolymerization of a (co)polymer synthesized from a monomer containing terephthalic acid.

The monomer composition for synthesizing recycled plastics of the one embodiment can be applied as a raw material for the preparation of monomers used in the synthesis of various recycled plastics (e.g., polyethylene terephthalate (PET), polybutylene terephthalate (PBT), polybutylene adipate terephthalate (PBAT), glycol-modified PET resin (glycol-modified polyethylene terephthalate, PETG), thermoplastic polyester elastomer (TPEE)), which will be described later, or can be used as a raw material for the preparation of other additives (e.g., dioctylterephthalate plasticizer) used in the processing of plastics (e.g., polyvinyl chloride (PVC)).

### 2. Method for preparing a monomer composition for synthesizing recycled plastic

According to another embodiment of the invention, there can be provided a method for preparing a monomer composition for synthesizing recycled plastic, comprising the steps of: subjecting a (co)polymer synthesized from a monomer containing terephthalic acid to a depolymerization reaction and removing a diol component; washing the depolymerization reaction product from which the diol component has been removed with a solvent at a temperature of 20°C or more and 100°C or less; and dissolving the washed depolymerization reaction product in water and then recrystallizing it, wherein the step of dissolving the washed depolymerization reaction product in water and then recrystallizing it comprises a step of dissolving the washed depolymerization reaction product in water at a temperature of 200°C to 300°C; and a step of cooling the dissolved solution to a temperature lower by 50°C to 200°C than the solution temperature.

The present inventors confirmed through experiments that similarly to the method for preparing the monomer composition for synthesizing recycled plastic of the other embodiments, since a washing step and a recrystallization step distinguished by the temperature section are applied in the step of recovering terephthalic acid from a (co)polymer synthesized from a monomer containing terephthalic acid, not only the ratio of isophthalic acid, which is other monomer and not terephthalic acid, which is the main synthetic target material in the present invention, is extremely reduced, but also the detected amount of organic impurities (acetic acid, formic acid) and aromatic impurities (4-carboxybenzaldehyde, benzoic acid, p-toluic acid) is extremely reduced, whereby, when synthesizing polyethylene terephthalate or high value-added plastics (PBT, TPEE) using this, the polymerization time is shortened, the polymerization production efficiency can be increased, and excellent physical properties can be realized in terms of color quality, and completed the present invention.

In particular, terephthalic acid recovered from a (co)polymer synthesized from a monomer containing terephthalic acid obtained by a conventional method usually contains 1 to 2% isophthalic acid as an impurity, whereas in the present invention, isophthalic acid or recrystallization organic solvent could be almost completely removed by the recrystallization step which are characteristic in the method for preparing the monomer composition for synthesizing recycled plastics, which will be described later.

This is because, in the step of dissolving the washed depolymerization reaction product in water and then recrystallizing it, the solubility of terephthalic acid is increased, and thus, crystals, or isophthalic acid or other impurities interposed between crystals can be dissolved and removed with a solvent to the maximum, and further, since the dissolved terephthalic acid has poor solubility relative to impurities, it can be easily precipitated into terephthalic acid crystals through the difference in solubility when the temperature is lowered subsequently.

Further, through the step of washing the depolymerization reaction product from which the diol component has been removed with a solvent at a temperature of 20°C or more and 100 °C or less, the acid component (HCl) and salt component remaining in the depolymerization reaction product from which the diol component has been removed can be washed and removed to the maximum, thereby suppressing reactor corrosion and increasing the yield and purity of terephthalic acid.

Specifically, the method for preparing the monomer composition for synthesizing recycled plastic of the other embodiment may include a step of subjecting a (co)polymer synthesized from a monomer containing terephthalic acid to a depolymerization reaction and removing a diol component.

The (co)polymer synthesized from the monomer containing terephthalic acid can be applied regardless of various forms and types, such as a (co)polymer synthesized from a monomer containing novel terephthalic acid produced through synthesis, a (co)polymer synthesized from a monomer containing recycled terephthalic acid produced through a recycling step; or a (co)polymer waste synthesized from monomers containing terephthalic acid, and the like.

However, if necessary, before proceeding with the depolymerization reaction of the (co)polymer synthesized from the monomer containing terephthalic acid, a pretreatment step of the (co)polymer synthesized from the monomer containing terephthalic acid can be carried out, thereby increasing the efficiency of the step of recovering terephthalic acid from the (co)polymer synthesized from the monomer containing terephthalic acid. Examples of the pretreatment step may include washing, drying, grinding, glycol decomposition, and the like. The specific method of each pretreatment process is not limited, and various methods widely used in the recovery step of terephthalic acid by depolymerization of a (co)polymer synthesized from a monomer containing terephthalic acid can be applied without limitation.

In the depolymerization reaction of the (co)polymer synthesized from the monomer containing terephthalic acid, the depolymerization reaction can be carried out under acidic, neutral, or basic conditions, and particularly, the depolymerization reaction may proceed under basic (alkaline) conditions. More specifically, the depolymerization reaction of the (co)polymer synthesized from the monomer containing terephthalic acid can be carried out in the presence of water, an alkylene glycol, or an alcohol solvent. Specific examples of the alkylene glycol solvent include ethylene glycol, and specific examples of the alcohol solvent include ethanol.

Further, the depolymerization reaction of the (co)polymer synthesized from the monomer containing terephthalic acid can be carried out under basic conditions. The type of the base is not particularly limited, and examples thereof include sodium hydroxide (NaOH).

The depolymerization reaction of the (co)polymer synthesized from the monomer containing terephthalic acid can be carried out by reacting a base in an amount of 2.3 mol or less, or 1 mol or more and 2.3 mol or less, or 1.5 mol or more and 2.3 mol or less relative to 1 mol of a (co)polymer synthesized from a monomer containing terephthalic acid.

In the depolymerization reaction of the (co)polymer synthesized from the monomer containing terephthalic acid, when the base is reacted in an amount of more than 2.3 mol relative to 1 mol of the (co)polymer synthesized from the monomer containing terephthalic acid, isophthalic acid and sodium (Na), which are impurities other than terephthalic acid, which are the main synthetic target materials, are increased due to the influence of the decrease in the solubility of isophthalate and the increase in the amount of alkaline salt generated, which makes it difficult to sufficiently remove it even by a washing step described later.

Further, the temperature at which the depolymerization reaction of the (co)polymer synthesized from the monomer containing terephthalic acid proceeds is not particularly limited, but for example, the reaction can proceed at a temperature of 25°C or more and 200°C or less, or 130°C or more and 180°C or less. In addition, the time required for the depolymerization reaction of the (co)polymer synthesized from the monomer containing terephthalic acid may be 0 minutes or more and 3 hours or less.

Meanwhile, after the (co)polymer synthesized from the monomer containing terephthalic acid is subjected to a depolymerization reaction, the diol component can be removed. For example, the polyethylene terephthalate (PET) depolymerization product includes ethylene glycol and terephthalate.

Since the main recovery target material of the present invention is terephthalic acid, other by-products can be removed through filtration. The filtered by-products can be recycled without a separation and purification process in the depolymerization reaction of the (co)polymer synthesized from the monomer containing terephthalic acid, or if necessary, can be recycled through separation and purification using conventional distillation, extraction, and adsorption methods.

Moreover, since the main recovery target material of the present invention is terephthalic acid, in the case of terephthalic acid salt, it can be converted to terephthalic acid through a neutralization process with an additional strong acid as described below.

As a specific example, the product of the depolymerization reaction of the (co)polymer synthesized from the monomer containing terephthalic acid is cooled to 50°C or less, and then ethylene glycol is removed through vacuum filtration to obtain terephthalate.

Meanwhile, the method for preparing the monomer composition for synthesizing recycled plastic of the other embodiment may include a step of washing the depolymerization reaction product from which the diol component has been removed with a solvent at a temperature of 20°C or more and 100°C or less.

As described above, in the depolymerization reaction product from which the diol component has been removed, ethylene glycol is removed, and terephthalic acid obtained by neutralization of terephthalic acid salt can be contained. However, since various impurities remain during the recovery step of obtaining terephthalic acid, washing can be performed in order to sufficiently remove them to secure high-purity terephthalic acid.

Specifically, the washing step may include a step of washing with a solvent at a temperature of 20°C or more and 100°C or less. The temperature condition means the temperature inside a washing container where washing with a solvent is performed, and various heating devices can be applied without limitation in order to maintain a high temperature out of room temperature. Thereby, the effect of minimizing corrosion of the reactor by a strong acid after the neutralization step can be exhibited.

The step of washing the depolymerization product from which the diol component has been removed with a solvent at a temperature of 20°C or more and 100°C or less can be repeated at least once or more. Further, if necessary, after the step of washing the depolymerization reaction product from which the diol component has been removed with a solvent at a temperature of 20°C or more and 100°C or less is performed, the step of removing the residual solvent through filtration may be further performed.

The solvent used in the washing step may be water. As the washing proceeds using water without an organic solvent, environmental friendliness can be enhanced. Further, when an organic solvent such as ethanol is used instead of the water, the salt (NaCl) cannot be effectively removed, and thus, a problem of increasing the residual amount of sodium (Na) in the composition may occur.

The solvent used in the washing step can be used in a weight ratio of 1 part by weight or more and 50 parts by weight or less, or 5 parts by weight to 10 parts by weight based on 1 part by weight of the (co)polymer synthesized from the monomer containing terephthalic acid used in the depolymerization reaction.

Meanwhile, the method for preparing a monomer composition for synthesizing recycled plastic according to another embodiment may include a step of dissolving the washed depolymerization reaction product in water and then recrystallizing it.

As described above, various impurities remain during the recovery step of obtaining terephthalic acid, recrystallization can proceed in order to sufficiently remove this and secure high-purity terephthalic acid.

Specifically, the recrystallization step may include a step of dissolving the washed depolymerization reaction product in water and then recrystallizing it. Through the step of dissolving the washed depolymerization reaction product in water and then recrystallizing it, the solubility of terephthalic acid or a salt thereof contained in the depolymerization reaction product is increased and thus, crystals, or impurities such as isophthalic acid, organic impurities, aromatic impurities interposed between crystals can be dissolved with a solvent to the maximum, and further, since the dissolved terephthalic acid has poor solubility relative to impurities, it can be easily precipitated into terephthalic acid crystals through the difference in solubility when the temperature is lowered subsequently.

More specifically, the step of dissolving the washed depolymerization reaction product in water and recrystallizing it may include a step of dissolving the washed depolymerization reaction product in water at a temperature of 200°C to 300°C, or 200°C to 250°C, or 205°C to 250°C; and a step of cooling the dissolved solution to a temperature lower by 50°C to 200°C than the solution temperature.

Despite the high temperature range and the cooling temperature range where recrystallization proceeds are relatively mild conditions, there is an advantage in that impurities can be removed at a high level and the terephthalic acid recovery efficiency can be increased. The temperature condition means the temperature inside the container in which recrystallization by the solvent proceeds, and various heating and cooling devices can be applied without limitation in order to maintain high and low temperatures outside the normal temperature.

Specific examples of the dissolution, cooling conditions, apparatus, and method of the recrystallization step are not particularly limited, and various methods widely used in the existing recrystallization technology field of terephthalic acid are applicable without limitation.

In the step of dissolving the washed depolymerization reaction product in water at a temperature of 200°C to 300°C, 1 part by weight to 60 parts by weight, or 10 parts by weight to 50 parts by weight of water can be used based on 1 part by weight of the (co)polymer synthesized from the monomer containing terephthalic acid. When water is used in an amount of less than 1 part by weight based on 1 part by weight of the (co)polymer synthesized from the monomer containing terephthalic acid, the temperature for dissolving the terephthalic acid contained in the washed depolymerization reaction product becomes too high, the process efficiency is deteriorated, and it is not easy to remove impurities through recrystallization. On the other hand, when water is used in an amount of more than 60 parts by weight based on 1 part by weight of a (co)polymer synthesized from a monomer containing terephthalic acid, the solubility of terephthalic acid contained in the washed depolymerization reaction product may become too high, and thus the yield of terephthalic acid recovered after recrystallization may decrease, and the process efficiency may decrease due to the use of a large amount of solvent.

More specifically, the step of dissolving the washed depolymerization reaction product in water at a temperature of 200°C to 300°C includes a step of dissolving the washed depolymerization reaction product in water at a temperature of 200°C to 220°C, wherein the content of the water may be 30 parts by weight to 60 parts by weight based on 1 part by weight of the (co)polymer synthesized from the monomer containing terephthalic acid.

Further, the step of dissolving the washed depolymerization reaction product in water at a temperature of 200°C to 300°C includes a step of dissolving the washed depolymerization reaction product in water at a temperature of 240°C to 300°C, wherein the content of the water may be 1 part by weight to 20 parts by weight, or 5 parts by weight to 20 parts by weight based on 1 part by weight of the (co)polymer synthesized from the monomer containing terephthalic acid.

On the other hand, the step of dissolving the washed depolymerization reaction product in water and then recrystallizing it may include a step of cooling the dissolved solution to a temperature lower by 50°C to 200°C than the solution temperature, after the step of dissolving the washed depolymerization reaction product in water at a temperature of 200°C to 300°C.

Through a difference in solubility between terephthalic acid and impurities due to the cooling, crystallized terephthalic acid is obtained by filtration or the like, and impurities can be removed in a state of being dissolved in the solvent.

The specific cooling conditions of the cooling step are not particularly limited, but for example, the dissolved solution can be cooled to a temperature lower by 50°C to 200°C than the solution temperature. That is, the difference between the solution temperature and the cooling temperature (difference value obtained by subtracting the cooling temperature from the solution temperature) may be 50°C to 200°C. Thus, terephthalic acid dissolved in a high-temperature solution can be precipitated and recovered due to the difference in solubility between the solution temperature and the cooling temperature.

The specific cooling conditions of the cooling step are not particularly limited, but for example, cooling can proceed at a temperature of 100°C or less, or 50°C or more and 100°C or less, or 80°C or more and 100°C or less. As for the specific drying device and method used during the cooling, various known cooling techniques can be applied without limitation.

Meanwhile the method for preparing the monomer composition for synthesizing recycled plastic of the other embodiment may further include a step of subjecting the depolymerization reaction product from which the diol component has been removed to a neutralization reaction with an acid, before the step of washing the depolymerization reaction product from which the diol component has been removed.

For example, polyethylene terephthalate (PET) alkaline decomposition products include ethylene glycol and terephthalate, but since the main recovery target material of the present invention is terephthalic acid, in the case of the terephthalate obtained by the alkaline decomposition, it can be converted to terephthalic acid through a further neutralization step with a strong acid. That is, when the depolymerization reaction of the (co)polymer synthesized from the monomer containing terephthalic acid is alkaline decomposition, it can undergo a neutralization reaction step with an acid.

The acid used in the neutralization reaction may be a strong acid, and examples thereof include hydrochloric acid (HCl). Due to the neutralization reaction by the strong acid, the pH may be 4 or less, or 2 or less at the time of completion of the neutralization reaction. The temperature during the neutralization reaction can be adjusted to 25°C or more and 100°C or less.

Further, if necessary, after proceeding the step of a neutralization reaction step by acid of the depolymerization reaction product from which the diol component has been removed, a step of removing residual impurities through filtration can be further performed.

Meanwhile, the method for preparing a monomer composition for synthesizing recycled plastic of the other embodiment may further include a step of purifying the depolymerization reaction product from which the diol component has been removed, after the step of subjecting a (co)polymer synthesized from a monomer containing terephthalic acid to a depolymerization reaction and removing a diol component.

Residual impurities can be removed through the purification, and specific purification conditions are not particularly limited. As for specific purification devices and methods, various purification techniques well-known conventionally can be applied without limitation.

In one specific example, the purification step of the depolymerization reaction product from which the diol component has been removed may include a step of dissolving and filtering the depolymerization reaction product from which the diol component has been removed; and an adsorption step through an adsorbent.

In the step of dissolving and filtering the depolymerization reaction product from which the diol component has been removed, water may be used as a solvent for dissolving the depolymerization reaction product from which the diol component has been removed, and the dissolution temperature may be 25°C or more and 100°C or less. Thereby, it is possible to remove the (co)polymer synthesized from the monomer containing residual terephthalic acid that did not react in the depolymerization reaction.

In the adsorption step through the adsorbent, activated carbon may be used as an example of the adsorbent.

In the purification step of the depolymerization reaction product from which the diol component has been removed, if necessary, an extraction step, a washing step, a precipitation step, a recrystallization step, a drying step, and the like can be further applied without limitation.

Further, as described above, in the case where the depolymerization reaction is an alkaline decomposition, if the purification step of the depolymerization reaction product from which the diol component has been removed is performed, after the purification step of the depolymerization reaction product from which the diol component has been removed, a step of subjecting the depolymerization reaction product from which the diol component has been removed to a neutralization reaction with an acid, and then a step of washing the depolymerization reaction product from which the diol component has been removed can be performed.

On the other hand, the method for preparing the monomer composition for synthesizing recycled plastic of the other embodiment may further include a drying step, after the step of washing the depolymerization reaction product from which the diol component has been removed. The residual solvent can be removed by the drying, and the specific drying conditions are not particularly limited, but for example, the drying can proceed at a temperature of 100°C or more and 150°C or less. As for specific drying devices and methods used during the drying, various known drying techniques can be applied without limitation.

### 3. Recycled Plastic

According to another embodiment of the invention, there can be provided a recycled plastic comprising a reaction product of the monomer composition for synthesizing recycled plastic of the one embodiment and a comonomer. The details of the monomer composition for synthesizing recycled plastic includes all the contents described above in the one embodiment.

Examples corresponding to the recycled plastic are not particularly limited, and various plastics synthesized using terephthalic acid as a monomer can be applied without limitation, and a more specific example may be a (co)polymer synthesized from a monomer containing terephthalic acid.

In the (co)polymer synthesized from the monomer containing terephthalic acid, the (co)polymer includes both a polymer or a copolymer, and collectively refers to a reaction product obtained through (co)polymerization of monomers. The (co)polymer may include all low molecular weight compounds, oligomers, and polymers depending on the molecular weight range.

The (co)polymer synthesized from the monomer containing terephthalic acid may include at least one (co)polymer selected from the group consisting of polyalkylene terephthalate, polyalkylene terephthalate-based copolymer, and thermoplastic polyester elastomer. That is, the (co)polymer synthesized from the monomer containing terephthalic acid may include one type of polyalkylene terephthalate, one type of polyalkylene terephthalate-based copolymer, one type of thermoplastic polyester elastomer, or a mixture of two or more thereof

The polyalkylene terephthalate-based copolymer means a copolymer obtained by further reacting an additional comonomer based on alkylene glycol and terephthalic acid which are monomers for synthesizing polyalkylene terephthalate.

Examples of the comonomer capable of reacting with high-purity terephthalic acid contained in the monomer composition for synthesizing recycled plastic of the one embodiment are also not particularly limited, and specific examples thereof include aliphatic diols, polyalkylene oxides, fatty acids, fatty acid derivatives, or combinations thereof.

As the aliphatic diol, for example, a diol having a number average molecular weight (Mn) of 300 g/mol or less, that is, at least one selected among ethylene glycol, propylene glycol, 1,2-propanediol, 1,3-propanediol, 1,4-butanediol (1,4-BG), 1,5-pentanediol, 1,6-hexanediol, and 1,4-cyclohexanedimethanol (1,4-CHDM) can be used, and as a specific example, 1,4-butanediol, ethylene glycol, 1,4-cyclohexanedimethanol, or a mixture thereof can be used.

The polyalkylene oxide is a unit constituting a soft fraction, and may include an aliphatic polyether as a constituent component. As an example, at least one selected among polyoxyethylene glycol, polypropylene glycol, poly(tetramethylene ether)glycol (PTMEG), polyoxyhexamethylene glycol, a copolymer of ethylene oxide and propylene oxide, an ethylene oxide addition polymer of polypropylene oxide glycol, a copolymer of ethylene oxide and tetrahydrofuran can be used, and as a specific example, PTMEG can be used. Particularly, PTMEG having a number average molecular weight (Mn) of 600 g/mol to 3,000 g/mol, 1,000 g/mol to 2,500 g/mol, or 1,500 g/mol to 2,200 g/mol can be used.

The fatty acid may be at least one type of aliphatic carboxylic acid compounds excluding terephthalic acid as an example, and adipic acid can be used as a specific example. The fatty acid derivative is a compound derived from the above-mentioned fatty acid, and as an example, at least one selected among fatty acid ester, fatty acid chloride, fatty acid anhydride, and fatty acid amide can be used, and as a specific example, adipic acid ester can be used.

In a more specific example, when 1,4-butanediol, which is the aliphatic diol, is used as a comonomer capable of reacting with the terephthalic acid, polybutylene terephthalate (PBT), which is a type of polyalkylene terephthalate, can be obtained by polymerization reaction of terephthalic acid and 1,4-butanediol.

Further, when ethylene glycol, which is an aliphatic diol, is used as a comonomer capable of reacting with high-purity terephthalic acid contained in the monomer composition for synthesizing recycled plastic of the one embodiment, polyethylene terephthalate (PET), which is a type of the polyalkylene terephthalate, can be obtained through a polymerization reaction of terephthalic acid and ethylene glycol.

Further, when 1,4-butanediol as the aliphatic diol and PTMEG as the polyalkylene oxide are used together as a comonomer capable of reacting with high-purity terephthalic acid contained in the monomer composition for synthesizing recycled plastic of the one embodiment, a thermoplastic polyester elastomer (TPEE) can be obtained through a polymerization reaction of terephthalic acid, 1,4-butanediol, and PTMEG.

Further, when 1,4-butanediol as the aliphatic diol and adipic acid as the fatty acid are used together as a comonomer capable of reacting with high-purity terephthalic acid contained in the monomer composition for synthesizing recycled plastic of the one embodiment, polybutylene adipate terephthalate (PBAT), which is a type of the polyalkylene terephthalate-based copolymer, may be obtained through a polymerization reaction of terephthalic acid, 1,4-butanediol, and adipic acid.

Further, when the aliphatic diol ethylene glycol and 1,4-cyclohexanedimethanol are used together as a comonomer capable of reacting with terephthalic acid of high purity contained in the monomer composition for synthesizing recycled plastic of the on embodiment, a glycol-modified PET resin (glycol-modified polyethylene terephthalate, PETG), which is a kind of the polyalkylene terephthalate-based copolymer, can be obtained through a polymerization reaction of terephthalic acid, ethylene glycol, and 1,4-cyclohexanedimethanol.

As a specific example, the polyalkylene terephthalate may include at least one (co)polymer selected among polybutylene terephthalate, polyethylene terephthalate, polycyclohexylene dimethylene terephthalate, polyethylene naphthalate, polybutylene naphthalate and polytrimethylene terephthalate.

Further, as a specific example, the polyalkylene terephthalate-based copolymer may include at least one (co)polymer selected from polybutylene adipate terephthalate (PBAT) and glycol-modified PET resin (glycol-modified polyethylene terephthalate, PETG).

Examples of the reaction method of the monomer composition for synthesizing recycled plastic and the comonomer are not particularly limited, and various known methods can be applied without limitation. However, specific examples of the reaction of the monomer composition for synthesizing recycled plastic and the comonomer may include melt-polycondensation and solid-state polymerization.

As a more specific example, when preparing a thermoplastic polyester elastomer (TPEE) from the recycled plastic, aromatic dicarboxylic acid, aliphatic diol and polyalkylene oxide is subjected to an esterification reaction in the presence of a titanium butoxide (TBT) catalyst at 180°C or more and 250°C or less for 30 minutes or more and 210 minutes or less to produce BHBT (bis(4-hydroxy)butyl terephthalate) oligomer, and then the TBT catalyst is re-charged, and the melt-polycondensation reaction may be carried out at 200°C or more and 270°C or less for 20 minutes or more and 240 minutes or less while gradually reducing the pressure from 760 torr to 0.3 torr. After the completion of the melt-polycondensation reaction, it may be discharged into the reactor under nitrogen pressure to pelletize the strands to form a pellet.

Then, solid-phase polymerization of the pellets in a solid-phase polymerization reactor or a rotatable vacuum dryer in a temperature range of 140°C or more and 200°C or less for 10 hours or more and 24 hours or less can be carried out under an inert air flow such as nitrogen under high vacuum.

Further, when producing polyalkylene terephthalate from the recycled plastic, an aromatic dicarboxylic acid and an aliphatic diol having a number average molecular weight (Mn) of 300 g/mol or less may be melt-polymerized and then solid-phase polymerized.

As a specific example, the polyalkylene terephthalate may be at least one selected from among polybutylene terephthalate, polyethylene terephthalate, polycyclohexylene dimethylene terephthalate, polyethylene naphthalate, polybutylene naphthalate and polytrimethylene terephthalate.

For the polyalkylene terephthalate resin, a low molecular weight pellet obtained by melt polymerization is placed in a solid-phase polymerization reactor, and reacted under high vacuum and inert conditions as presented in the solid-phase polymerization of the thermoplastic polyester elastomer (TPEE) described above, thereby obtaining a high molecular weight resin.

Due to the reaction of the monomer composition for synthesizing recycled plastic and the comonomer, the specific method for preparing the polybutylene terephthalate, polyethylene terephthalate, polycyclohexylene dimethylene terephthalate, polyethylene naphthalate, polybutylene naphthalate, polytrimethylene terephthalate, or polybutylene adipate terephthalate is not particularly limited, and various processes widely known in in the field of the existing recycled plastic synthesis can be applied without limitation.

The recycled plastic may have a yellow index of 50 or less, or 5 or less, or 0.5 or more, or 0.5 to 50, or 0.5 to 5.

When the yellow index of the recycled plastic is excessively increased to more than 50, the color characteristics are deteriorated due to the yellowing phenomenon of the recycled plastic. Examples of the method for measuring the yellow index of the recycled plastic are not particularly limited, and various methods for measuring color characteristics in the plastic field can be applied without limitation.

However, in an example of a method of measuring the yellow index of the recycled plastic, measurement can be performed in a reflection mode using a HunterLab UltraScan PRO Spectrophotometer device.

The recycled plastic may have a color coordinate b* value of 3 or less, or 2 or less, or 1 or less, or 0.1 or more, or 0.5 or more, or 0.9 or more, or 0.1 to 3, or 0.1 to 2, or 0.1 to 1, or 0.5 to 3, or 0.5 to 2, or 0.5 to 1, or 0.9 to 3, or 0.9 to 2, or 0.9 to 1.

Further, the recycled plastic may have a color coordinate L* value of 84.5 or more, or 85 or more, or 86 or less, or 84.5 to 86, or 85 to 86.

The recycled plastic has a color coordinate a* value of -0.3 or less, or -0.4 or less, or -0.5 or less, or -0.6 or less, or -1 or more, or -1 to -0.3, or -1 to -0.4, or -1 to -0.5, or -1 to -0.6.

When the color coordinate b* value of the recycled plastic is excessively increased to more than 3, the color characteristics are deteriorated due to the yellowing phenomenon of the recycled plastic. Examples of the method for measuring the color coordinates L*, a*, and b* values of the recycled plastic are not particularly limited, and various methods for measuring color characteristics in the plastics field can be applied without limitation.

However, in an example of a method of measuring the color coordinates L*, a*, and b* values of the recycled plastic, measurements can be performed in a reflection mode using a HunterLab UltraScan PRO Spectrophotometer device.

The recycled plastic may have a melt flow index of 5 g/lOmin to 30 g/lOmin, or 15 g/lOmin to 25 g/lOmin as measured by ASTM D1238. When the melt flow index of the recycled plastic increases to more than 30 g/10 min, molding of the recycled plastic is not easy.

However, in an example of the method for measuring the melt flow index of the recycled plastic, the melt flow index can be measured at 230°C to 250° and a load of 2.16 kg for 4 minutes according to ASTM D1238.

More specifically, for example, in the case of a thermoplastic polyester elastomer (TPEE), the melt flow index can be measured at 230°C and a load of 2.16 kg for 4 minutes according to ASTM D1238. Further, in the case of the polyalkylene terephthalate, the melt flow index can be measured at 250°C and a load of 2.16 kg for 4 minutes according toASTMD1238.

### 4. Molded Product

According to another embodiment of the invention, a molded article comprising the recycled plastic of the other embodiment can be provided. The details of the recycled plastic includes all the contents described above in the other embodiments.

The molded article can be obtained by applying the recycled plastic to various known plastic molding methods without limitation. As an example of the molding method, injection molding, foam injection molding, blow molding, or extrusion molding may be mentioned.

Examples of the molded article are not particularly limited, and can be applied to various molded articles using plastic without limitation. Examples of the molded article include automobiles, electrical and electronic products, communication products, and daily necessities.

### 5. Plasticizer Composition

According to another embodiment of the invention, there can be provided a plasticizer composition comprising a reaction product of the monomer composition for synthesizing recycled plastic of the one embodiment and an alcohol. The details of the monomer composition for synthesizing the recycled plastic includes all the contents described above in the one embodiment.

Generally, a plasticizer is obtained by appropriately adding a resin such as polyvinyl chloride (PVC) and various additives such as a filler, a stabilizer, a pigment, and an antifogging agent to impart various processed physical properties, and is used as materials for a variety of products, ranging from wires, pipes, flooring, wallpaper, sheets, artificial leather, tarpaulin, tapes, and food packaging materials by processing methods such as extrusion molding, injection molding, and calendaring.

Typically, for the plasticizer, the alcohol reacts with polycarboxylic acid such as phthalic acid and adipic acid to form the corresponding ester. In addition, in consideration of domestic and international regulations on phthalate-based plasticizers that are harmful to the human body, research on plasticizer compositions that can replace phthalate-based plasticizers such as terephthalate-based, trimellitate-based, and other polymer-based plasticizers is being continued.

The reaction product of the monomer composition for synthesizing recycled plastic of the one embodiment and alcohol may include a terephthalate-based compound. Specifically, a terephthalate-based compound can be obtained through a direct esterification reaction between terephthalic acid and alcohol contained in the monomer composition for synthesizing recycled plastic of the one embodiment.

The direct esterification reaction may comprise the steps of adding terephthalic acid to alcohol, then adding a catalyst and reacting the mixture under a nitrogen atmosphere; removing unreacted alcohol and neutralizing unreacted acid; and dehydrating and filtrating the result by distillation under reduced pressure.

Examples of the terephthalate-based compound are not particularly limited, but examples thereof include dioctyl terephthalate (DOTP), diisononyl terephthalate (DINTP), diisodecyl terephthalate (DIDTP), or di(2-propylheptyl)terephthalate (DPHTP) and the like.

The terephthalate-based compound can be prepared through a direct esterification reaction in which any one alcohol selected from the group consisting of octanol, isononyl alcohol, isodecyl alcohol and 2-propylheptyl alcohol, and terephthalic acid are reacted.

The plasticizer composition can be applied to the manufacture of electric wires, flooring materials, automobile interior materials, films, sheets, wallpaper or tubes.

### [Advantageous Effects]

According to the present invention, a monomer composition for synthesizing recycled plastic that is capable of remarkably reducing the content of organic impurities and thus securing high-purity terephthalic acid, when recovering terephthalic acid through a depolymerization reaction of a (co)polymer synthesized from a monomer containing terephthalic acid, a method for preparing the same, and a recycled plastic, molded product, and plasticizer composition using the same can be provided.

### [DETAILED DESCRIPTION OF THE EMBODIMENTS]

Hereinafter, the present invention will be explained in detail with reference to the following examples. However, these examples are for illustrative purposes only, and the scope of the present invention is not limited thereto.

### <EXAMPLE>

### Example 1

### (1) Preparation of recycled terephthalic acid monomer composition

192 g (1 mol) of polyethylene terephthalate (PET) bottle scrap, 1250 g (about 20 mol) of ethylene glycol (EG), and 82 g (2.05 mol) of sodium hydroxide (NaOH) were placed in a 3L SUS reactor, and then stirred at 180°C for 2 hours in a closed system to proceed a PET depolymerization reaction. The product of the depolymerization reaction was cooled to 20~30°C, and then filtered under vacuum to obtain sodium terephthalate (Na₂-TPA).

The filtrate containing sodium terephthalate (Na₂-TPA) was completely dissolved in water, and then filtered under vacuum again to remove unreacted PET and other solid impurities. Then, the filtrate was purified through activated carbon adsorption, and then neutralized using 6M HCl at 20~30°C. The slurry lowered to pH 2 or less was filtered under vacuum again to obtain terephthalic acid (TPA).

In order to remove NaCl generated during the neutralization step, washing was performed at 20~30°C using 1920 g of water, which is 10 times the mass of PET used, and filtered under vacuum.

Then, 9600 g of water, which is 50 times the mass of PET used, was added, heated to 205°C, completely dissolved at 205°C, and then the TPA aqueous solution was cooled to 100°C or less and normal pressure to recrystallize terephthalic acid. The obtained slurry was filtered under vacuum at 20~30°C to recover terephthalic acid (TPA) crystals.

Then, drying was performed in a convection oven at 120°C to recover the recycled terephthalic acid (TPA), thereby preparing a recycled terephthalic acid monomer composition.

### (2) Preparation of recycled plastic

200 g of the recycled terephthalic acid monomer composition obtained in Example 1(1), 200 g of 1,4-butylene glycol, and 125 g of poly(tetramethylene) glycol (PTMEG) having a number average molecular weight of 1,000~2,000 g/mol was placed in an ester interchange reactor, and then 0. 1wt% of TBT catalyst was added thereto. The mixture was reacted for 120-180 minutes while maintaining 200~240°C, and the reaction was terminated when the reaction rate (the value obtained by converting the amount of water, which is the reaction effluent, into the reaction rate) was 90% or more to obtain an oligomer.

Then, the prepared oligomer was transferred to a polycondensation reactor, and 0.1wt% of TBT catalyst, 0.14-0.15wt% of hindered phenolic antioxidant, and 0.15~0.2wt% of aromatic amine antioxidant or sulfur-based stabilizer were added thereto. The pressure was reduced from 760 torr to 0.3 torr for 30 minutes while maintaining 230~250°C, a melt polycondensation reaction was carried out, and then melt polycondensation was carried out under high vacuum conditions of 0.3 torr or less until the torque applied to the stirrer reached the desired torque. After completion of the reaction, it was discharged using nitrogen pressure, stranded, cooled, and then pelletized to prepare a thermoplastic polyester elastomer (TPEE) resin.

### Example 2

### (1) Preparation of recycled terephthalic acid monomer composition

192 g (1 mol) of polyethylene terephthalate (PET) bottle scrap, 1600 g of water and 82 g (2.05 mol) of sodium hydroxide (NaOH) were placed in a 3L SUS reactor, and then stirred at 180°C for 2 hours in a closed system to proceed a PET depolymerization reaction. The product of the depolymerization reaction was cooled to 50°C or less, and then filtered under vacuum to obtain unreacted PET and other solid impurities.

Then, the filtrate was purified through activated carbon adsorption, and then neutralized using 6M HCl at 20 ~ 30°C. The slurry lowered to pH 2 or less was filtered under vacuum again to obtain terephthalic acid (TPA).

In order to remove NaCl generated during the neutralization step, washing was performed at 20~30°C using 1920 g of water, which is 10 times the mass of PET used, and filtered under vacuum.

Then, 1920 g of water, which is 10 times the mass of PET used, was added thereto, heated to 250°C, completely dissolved at 250°C, and then the TPA aqueous solution was cooled to 100°C or less and normal pressure to recrystallize terephthalic acid. The obtained slurry was then filtered under vacuum at 20~30°C to recover terephthalic acid (TPA) crystals.

Then, drying was performed in a convection oven at 120°C to recover the recycled terephthalic acid (TPA), thereby preparing a recycled terephthalic acid monomer composition.

### (2) Preparation of recycled plastic

A thermoplastic polyester elastomer (TPEE) resin was prepared in the same manner as in Example 1(2), except that the recycled terephthalic acid monomer composition obtained in Example 2(1) was used instead of the recycled terephthalic acid monomer composition obtained in Example 1(1).

### Example 3

### (1) Preparation of recycled terephthalic acid monomer composition

A recycled terephthalic acid monomer composition was prepared in the same manner as in Example 1(1).

### (2) Preparation of recycled plastic

300 g of the recycled terephthalic acid monomer composition obtained in Example 1(1) and 300 g of 1,4-butylene glycol were put into an esterification reactor, and 0.1 wt% of a TBT catalyst was added thereto. The mixture was reacted while maintaining 200-240°C 120~180 minutes, and the reaction was terminated when the reaction rate (when the amount of water, which is the reaction effluent, converted to the reaction rate) was 90% or more to obtain an oligomer.

Then, the prepared oligomer was transferred to a polycondensation reactor, the pressure was reduced from 760 torr to 0.3 torr for 30 minutes while maintaining 230~260°C, and the melt polycondensation was carried out, and the melt polycondensation reaction was carried out under high vacuum conditions of 0.3 torr or less until the torque applied to the stirrer reached a desired torque value. After completion of the reaction, it was discharged using nitrogen pressure, stranded, cooled and then pelletized to prepare a polybutylene terephthalate (PBT) resin.

### <COMPARATIVE EXAMPLE>

### Comparative Example 1

A recycled terephthalic acid monomer composition and a recycled plastic were prepared in the same manner as in Example 1, except that as shown in Table 1 below, the recrystallization step of Example 1(1) was not performed, and secondary washing was performed with 3840 g of water, which is 20 times the mass of PET used, at a temperature of 20~30°C.

### <REFERENCE EXAMPLE>

### Reference Example 1

A plastic was prepared in the same manner as in Example 1, except that a commercially available TPA reagent (manufacturer: TCI) was used instead of the recycled terephthalic acid monomer composition.

### <Experimental Example 1>

The physical properties of the recycled terephthalic acid monomer compositions obtained in Examples and Comparative Examples were measured by the following methods, and the results are shown in Table 1 below.

### 1. Content of terephthalic acid (TPA) and isophthalic acid (IPA)

5~20 mg of recycled terephthalic acid monomer composition was collected as a sample under the conditions of normal pressure and 20~30°C, dissolved in 1 ml DMSO-d6 solvent, and then ¹H NMR spectrum was obtained through an Agilent DD1 500 MHz NMR instrument. All material peaks detected, such as terephthalic acid (TPA) and isophthalic acid (IPA), were respectively assigned and integrated using an analysis software (MestReC). The molar ratio (mol%) of terephthalic acid and isophthalic acid contained in 100 mol% of all monomer compounds analyzed in the sample was calculated based on the peak integral value.

### 2. Content of formic acid (FA) and acetic acid (AA)

0.5 g of a recycled terephthalic acid monomer composition was collected as a sample under the conditions of normal pressure and 20~30°C, and analyzed through a Headspace-GC/MS (oven temperature: 150°C) device, and the weight ratio (*µ*g/g) of formic acid (FA), acetic acid (AA) contained therein was measured based on 1 g of the sample.

### 3. Contents of 4-carboxybenzaldehyde (4-CBA), benzoic acid (BZA), and p-toluic acid (p-TA)

0.5 g of the recycled terephthalic acid monomer composition was collected as a sample under the conditions of normal pressure and 20~30°C , and analyzed through a liquid chromatography (LC) device, and weight ratio (*µ*g/g) of 4-carboxybenzaldehyde (4-CBA), benzoic acid (BZA) and p-toluic acid (p-TA) contained therein was measured based on 1 g of the sample. The analysis limit (lower limit) for each substance using LC equipment was 20 *µ*g/g for 4-CBA, 80 *µ*g/g for BZA, and 100 *µ*g/g for p-TA, respectively, and the case where it was smaller than the analysis limit value and thus was not detected was denoted as N.D (Not detected).

**[Table 1]**

| Measurement result of Experimental Example 1 | | | | | | | | | | |
|---|---|---|---|---|---|---|---|---|---|---|
| Category | Depolymeriz ation solvent | Washing condition | Recrystalliza tion condition | TPA (mol %) | IPA (mol %) | FA ( *µ*g /g) | AA (*µ*g /g) | 4-CB A (*µ*g /g) | BZ A (*µ*g /g) | p-T A ( *µ*g /g) |
| Example 1 | Ethylene glycol | 20∼30°C /water (10 times relative to PET mass) | [Dissolved]2 05°C / water (50 times relative to PET mass) [cooled] 100°C or less, normal pressure | 100 | 0 | 4.3 | 0.6 | N. D | N. D | N. D |
| Example 2 | Water | 20∼30°C / water (10 times relative to PET mass) | [dissolved] 250°C / water (100 times relative to PET mass) [cooled] 100°C or less, normal pressure | 99.6 | 0.4 | 4.0 | 0.5 | N. D | N. D | N. D |
| Compara tive Example 1 | Ethylene glycol | [primary] 20 ∼30°C / water (10 times relative to PET mass)[secon dary] 20∼30°C / water (20 times relative to PET mass) | - | 99.1 | 0.9 | 13. 5 | 2.0 | N. D | N. D | N. D |
| Referenc e Example 1 | - | - | - | 100 | 0 | 1.9 | 299 .6 | 153 | N. D | 13 6 |

As shown in Table 1, terephthalic acid was contained at 100 mol% in the monomers including in the recycled terephthalic acid monomer composition obtained in Example 1, and terephthalic acid was contained at 99.6 mol% in the monomers included in the recycled terephthalic acid monomer composition obtained in Example 2, so that a considerable amount of the impurity isophthalic acid was removed. On the other hand, it could be confirmed that in the monomers contained in the recycled terephthalic acid monomer composition obtained in Comparative Example 1, isophthalic acid as impurities was contained in an excess of 0.9 mol% compared to Examples, so that the purification efficiency of terephthalic acid was greatly improved.

On the other hand, in the recycled terephthalic acid monomer composition obtained in Examples 1 and 2, formic acid (FA) as other impurities was detected at 4.0 *µ*g/g to 4.3 *µ*g/g, and acetic acid (AA) was detected at 0.5 *µ*g/g to 0.6 *µ*g/g, and 4-carboxybenzaldehyde (4-CBA), benzoic acid (BZA), and p-toluic acid (p-TA) were not detected, thus showing excellent purification efficiency for impurities.

On the other hand, it could be confirmed that in the recycled terephthalic acid monomer composition obtained in Comparative Example 1, formic acid (FA) as other impurities was detected at 13.5 *µ*g/g, and acetic acid (AA) was detected at 2.0 *µ*g/g, which was excessive compared to Examples, so that the purification efficiency for impurities were deteriorated.

In addition, it could be confirmed that in the commercially available TPA reagent of Reference Example 1 (manufacturer: TCI), acetic acid (AA), which is other impurity, was detected at 299.6 *µ*g/g, and 4-carboxybenzaldehyde (4-CBA) was detected at 153 *µ*g/g , and p-toluic acid (p-TA) was detected at 136*µ*g/g, which was excessive compared to Examples, resulting in poor purification efficiency for impurities.

### <Experimental Example 2>

The physical properties of the recycled plastics obtained in Examples and Comparative Examples were measured by the following methods, and the results are shown in Table 2 below.

### 1. Polymerization time

During the preparation of the thermoplastic polyester elastomer (TPEE) obtained in Examples and Comparative Examples, the time taken from the time when the oligomer was transferred to the polycondensation reactor and the pressure started to decrease until the target torque was reached was measured, and this time was evaluated as the polymerization time.

### 2. Melt Flow Index (MFI)

The melt flow index of the thermoplastic polyester elastomers (TPEE) obtained in Examples and Comparative Examples was measured at 230°C and a load of 2.16 kg for 4 minutes according to ASTM D1238.

### 3. Yellow index (YI), Color coordinates (L*, a*, b*)

An injection specimen (1.5T) was prepared from the thermoplastic polyester elastomer (TPEE) obtained in Examples and Comparative Examples, and the specimen was analyzed in a reflection mode using a HunterLab UltraScan PRO Spectrophotometer device.

**[Table 2]**

| | Measurement result of Experimental Example 2 | | | | | | | | |
|---|---|---|---|---|---|---|---|---|---|
| Category | Depolymeriz ation solvent | Washing condition | Recrystalliz ation condition | Polymeriza tion time (min) | MFI (g/10m in) | Y I | L* | a* | b * |
| Example 1 | Ethylene glycol | 20∼30°C / water (10 times relative to PET mass) | [Dissolved] 205°C/water (50 times relative to PET mass) [cooled] 100°C or less, normal pressure | 122 | 24 | 3. 0 | 85. 0 | - 0. 6 | 1. 7 |
| Example 2 | Water | 20∼30°C/wat er(10 times relative to PET mass) | [dissolved] 250°C / water (10 times relative to PET mass) [cooled] 100°C or less, normal pressure | 127 | 23 | 1. 1 | 85. 0 | - 0. 6 | 0. 9 |
| Compara tive Example 1 | Ethylene glycol | [primary] 20 ∼30°C / water (10 times relative to PET mass) [secondary] 20∼30°C/wat er (20 times relative to PET mass) | - | 183 | 24 | 8. 7 | 83. 5 | 0. 0 | 4. 8 |
| Referenc e Exampl e 1 | - | - | - | 129 | 22 | 6. 2 | 85. 0 | 0. 0 | 3. 4 |

As shown in Table 2, in the case of the TPEE (co)polymer synthesized from the recycled terephthalic acid monomer composition obtained in Examples 1 and 2, the polymerization time is as short as 122 minutes to 127 minutes, the melt flow index is 23 g/10 min to 24 g/10 min, the yellow index (YI) is 1.1 to 3.0, and the color coordinates are L* = 85.0, a* = -0.6 and b* = 0.9 to 1.7, showing excellent color quality.

On the other hand, in the case of the TPEE (co)polymer synthesized from the recycled terephthalic acid monomer composition obtained in Comparative Example 1, the polymerization time is 183 minutes, which is longer than in Examples, the yellow index (YI) is 8.7, the color coordinates are L*= 83.5, a* = 0, and b*= 4.8, showing poor color quality.

In addition, in the case of a TPEE (co)polymer synthesized from the commercially available TPA reagent (manufacturer: TCI) of Reference Example 1, the polymerization time is 129 minutes, which is longer than in Example, the yellow index (YI) is 6.2, the color coordinates are L* = 85, a* =0, and b* = 3.4, showing poor color quality.

## Claims

1. A monomer composition for synthesizing recycled plastic,
which comprises terephthalic acid, and
which further comprises one or more organic impurities selected from the group consisting of formic acid and acetic acid,
wherein a weight ratio of the organic impurities measured using a gas chromatography mass spectrometry is less than 5 ug relative to 1 g of the monomer composition for synthesizing recycled plastic, and
wherein the monomer composition for synthesizing recycled plastic is recovered from a (co)polymer synthesized from a monomer containing terephthalic acid.

2. The monomer composition for synthesizing recycled plastic according to claim 1, wherein:
further comprising an isophthalic acid having a molar ratio of less than 0.85 mol% based on 100 mol% of the total monomer compounds contained in the monomer composition for synthesizing recycled plastic.

3. The monomer composition for synthesizing recycled plastic according to claim 1, wherein:
the terephthalic acid is recovered from the (co)polymer that is synthesized from the monomer containing terephthalic acid used for the recovery of the monomer composition for synthesizing recycled plastic.

4. The monomer composition for synthesizing recycled plastic according to claim 1, wherein:
the terephthalic acid has a molar ratio of more than 99.15 mol% based on 100 mol% of the total monomer compounds contained in the monomer composition for synthesizing recycled plastic.

5. The monomer composition for synthesizing recycled plastic according to claim 1,
further comprising one or more aromatic impurities selected from the group consisting of 4-carboxybenzaldehyde, benzoic acid, and p-toluic acid,
wherein a weight ratio of the aromatic impurities measured using a liquid chromatography (LC) is less than 100 ug relative to 1 g of the monomer composition for synthesizing recycled plastic.

6. The monomer composition for synthesizing recycled plastic according to claim 1, wherein:
the (co)polymer synthesized from the monomer containing terephthalic acid comprises at least one (co)polymer selected from the group consisting of polyalkylene terephthalate, polyalkylene terephthalate-based copolymer, and thermoplastic polyester elastomer.

7. A method for preparing a monomer composition for synthesizing recycled plastic, comprising the steps of:
subjecting a (co)polymer synthesized from a monomer containing terephthalic acid to a depolymerization reaction and removing a diol component;
washing a depolymerization reaction product from which the diol component has been removed with a solvent at a temperature of 20°C or more and 100°C or less; and
dissolving the washed depolymerization reaction product in water and then recrystallizing it,
wherein the step of dissolving the washed depolymerization reaction product in water and then recrystallizing it comprises,
dissolving the washed depolymerization reaction product in water at a temperature of 200°C to 300°C; and
cooling a solution in which the depolymerization reaction product is dissolved to a temperature lower by 50°C to 200°C than the solution temperature.

8. The method for preparing a monomer composition for synthesizing recycled plastic according to claim 7, wherein:
the solvent of the step of washing the depolymerization reaction product from which the diol component has been removed with a solvent at a temperature of 20°C or more and 100°C or less is water.

9. The method for preparing a monomer composition for synthesizing recycled plastic according to claim 7, wherein:
in the washing step, 1 part by weight to 50 parts by weight of the solvent is used based on 1 part by weight of the (co)polymer synthesized from the monomer containing terephthalic acid.

10. The method for preparing a monomer composition for synthesizing recycled plastic according to claim 7, wherein:
in the step of dissolving the washed depolymerization reaction product in water at a temperature of 200°C to 300°C,
1 part by weight to 60 parts by weight of water is used based on 1 part by weight of the (co)polymer synthesized from the monomer containing terephthalic acid.

11. The method for preparing a monomer composition for synthesizing recycled plastic according to claim 7, wherein:
the step of dissolving the washed depolymerization reaction product in water at a temperature of 200°C to 300°C comprises,
dissolving the washed depolymerization reaction product in water at a temperature of 200°C to 220°C,
wherein the content of water is 30 parts by weight to 60 parts by weight based on 1 part by weight of the (co)polymer synthesized from the monomer containing terephthalic acid.

12. The method for preparing a monomer composition for synthesizing recycled plastic according to claim 7, wherein:
the step of dissolving the washed depolymerization reaction product in water at a temperature of 200°C to 300°C comprises,
dissolving the washed depolymerization reaction product in water at a temperature of 240°C to 300°C,
wherein the content of water is 1 part by weight to 20 parts by weight based on 1 part by weight of the (co)polymer synthesized from the monomer containing terephthalic acid.

13. The method for preparing a monomer composition for synthesizing recycled plastic according to claim 7, wherein:
the depolymerization reaction of the (co)polymer synthesized from a monomer containing terephthalic acid is carried out by reacting a base in an amount of 2.3 moles or less relative to 1 mole of the (co)polymer synthesized from the monomer containing terephthalic acid.

14. The method for preparing a monomer composition for synthesizing recycled plastic according to claim 7, wherein:
the depolymerization reaction of the (co)polymer synthesized from the monomer containing terephthalic acid is carried out in the presence of water or alkylene glycol solvent.

15. The method for preparing a monomer composition for synthesizing recycled plastic according to claim 7, wherein:
before the step of washing the depolymerization reaction product from which the diol component has been removed,
the method further comprises subjecting the depolymerization reaction product from which the diol component has been removed to a neutralization reaction with an acid.

16. A recycled plastic comprising a reaction product of the monomer composition for synthesizing recycled plastic of claim 1 and a comonomer.

17. The recycled plastic according to claim 16, wherein:
the recycled plastic has a yellow index of 50 or less.

18. The recycled plastic according to claim 16, wherein:
the recycled plastic has a color coordinate b* value of 3 or less.

19. A molded product comprising the recycled plastic of claim 16.

20. A plasticizer composition comprising a reaction product of the monomer composition for synthesizing recycled plastic of claim 1 and an alcohol.
